**Europäisches Patentamt**

**European Patent Office**

**Office européen des brevets**

(19)

(11) Veröffentlichungsnummer: **0 306 848 B1**

(12)

# EUROPÄISCHE PATENTSCHRIFT

(45) Veröffentlichungstag der Patentschrift: **03.11.93**

(51) Int. Cl.5: **C12N 15/00**, C12N 15/85

(21) Anmeldenummer: **88114317.6**

(22) Anmeldetag: **02.09.88**

(54) Expressionsvektor und Verfahren zur Expression von heterologen Proteinen in Säugerzellen.

(30) Priorität: **09.09.87 DE 3730246**

(43) Veröffentlichungstag der Anmeldung:
**15.03.89 Patentblatt 89/11**

(45) Bekanntmachung des Hinweises auf die
Patenterteilung:
**03.11.93 Patentblatt 93/44**

(84) Benannte Vertragsstaaten:
**AT BE CH DE ES FR GB GR IT LI LU NL SE**

(56) Entgegenhaltungen:
**DE-A- 3 545 126**

**NATURE, Band 303, 2. Juni 1983, (N.Y., London), U. WEIDLE et al. "The 5'flanking region of a human IFN-a mediates viral induction of transcription", S. 442-446**

(73) Patentinhaber: **BOEHRINGER MANNHEIM GMBH**

**D-68298 Mannheim(DE)**

(72) Erfinder: **Grummt, Friedrich, Prof. Dr.
Gegenbaurstrasse 1
D-8700 Würzburg(DE)**
Erfinder: **Weidle, Ulrich, Dr. rer. nat.
Landwehrstrasse 56
D-8000 München 2(DE)**

**Beschreibung**

Die Erfindung betrifft einen Expressionsvektor für Säugerzellen und ein Verfahren zur Expression von heterologen Proteinen in Säugerzellen. Die Expression von heterologen Proteinen in Säugerzellen ist zur Herstellung von therapeutischen Humanproteinen von großer Bedeutung. Im Gegensatz zur Expression in Prokaryonten oder Hefen können hierbei die Proteine in einer Form erhalten, die weitgehend ihrem natürlichen Aufbau (z. B. Glycosylierung) entspricht.

Üblicherweise ist ein derartiger Vektor ein Shuttle-Vektor und besteht aus einem bakteriellen Anteil mit einem bakteriellen Origin, der die Klonierung in Bakterien ermöglicht, einem Säugerorigin, der die Expression in Säugerzellen ermöglicht, einem Selektionssystem, durch das die Zellen ohne den Vektor abgetötet werden bzw. sich nicht weiter vermehren, dem Gen für das heterologe Protein sowie für seine Expression geeignete Promotor- und Terminatorsequenzen.

Bisher sind allerdings nur wenig Expressionssysteme in Säugerzellen bekannt. Beispiele hierfür sind bovine papilloma virus (BPV/Mäuse-Fibroblastenzellen) (P.N.A.S. USA 80 (1983) 397 - 401, EMBO J. 4 (1985) 91 - 103) sowie SV40/CHO (DHFR⁻) (Mol. Cell. Biol. 4 (1984) 166 - 172, DNA 3 (1984) 297 - 308). Beide Systeme haben wesentliche Nachteile. Das BPV-System ist allein auf Mäuse-Fibroblasten beschränkt. Außerdem ist die Replikationsrate der Vektoren nur sehr gering, so daß nur sehr wenig rekombinantes Protein erhalten werden kann. So konnte beispielsweise mit der Expressions-Kassette des Vektors pKCR-tPA$_1$ im BPV-System (DE 3545126) mit den besten Klonen lediglich eine Ausbeute von 1 $\mu$g tPA/$10^6$ Zellen/24 h/ml erhalten werden. Im CHO-System (DHFR⁻) konnte mit der gleichen Expressionskassette in einem das DHFR-Gen enthaltenden Vektor dagegen mit den besten Klonen eine Ausbeute von 15 - 40 $\mu$g tPA/$10^6$ Zellen/24 h/ml erhalten werden. Das CHO-System hat jedoch den Nachteil, daß die Selektion der optimalen Klone sehr aufwendig ist. So müssen, bis die besten Klone erhalten werden, mindestens 5 - 10 Selektionsschritte mit steigender Methotrexatkonzentration durchgeführt werden (Mol. and Cell Biol. 5 (1985) 1750 - 1759). Diese Selektion dauert üblicherweise ca. 6 - 9 Monate pro Klon.

Außerdem enthalten die bekannten Vektoren als Säuger-Origin-Sequenzen Sequenzen, die aus Viren (z. B. SV40 oder Cytomegalo) stammen. Da die Gegenwart von viralen Origin-Sequenzen bei der Herstellung von therapeutisch anzuwendenden Proteinen problematisch erscheint, besteht ein Bedarf an Vektoren, welche zur Expression in Säugerzellen geeignet sind, jedoch keine viralen Origin-Sequenzen mehr enthalten.

Der Erfindung liegt daher die Aufgabe zugrunde, diese Nachteile zu beseitigen und ein Expressionssystem zur Verfügung zu stellen, welches schnell amplifizierbar ist, eine Selektion der besten Klone in kurzer Zeit erlaubt, universell in Säugerzellen einsetzbar und von viralen Origin-Sequenzen frei ist.

Diese Aufgabe wird durch einen Vektor zur Expression von heterologen Proteinen in Säugerzellen gelöst, der dadurch gekennzeichnet ist, daß er mindestens eine erste Consensussequenz, welche 10 bis 12 Nukleotide und folgende Sequenz aufweist:

CTCTGAGATC,

CTCTAAGAGGAA oder

CTC$^A_T$ GAGA$^{GG}_{CC}$ AA

und eine zweite Consensussequenz, welche 9 bis 12 Nukleotide aufweist, wovon mindestens 9 Adenin und/oder Thymidin sind, und ein Bindeprotein binden kann, sowie die folgende Nukleotidfolge aufweist:

TGG(N)$_{6-7}$GCCAA,

wobei der Abstand zwischen erster und zweiter Consensussequenz 20 bis 150 Basenpaare beträgt, vor der ersten oder hinter der zweiten Consensussequenz eine DNA-Sequenz, welche für ein heterologes Protein kodiert, aufweist und ein ineffizientes Selektionssystem enthält. DNA-Fragmente, welche erfindungsgemäß mindestens eine erste und zweite Consensussequenz enthalten, werden im weiteren auch als muARS (murine autonomously replication Sequences) bezeichnet.

Vorzugsweise wird ein Vektor verwendet, der mindestens eine erste Consensussequenz, die homolog zu der Sequenz

CTC$^A_T$ GAGA$^{GG}_{CC}$ AA

ist und eine zweite, zur Bindung an ein Bindeprotein befähigte Consensussequenz, die homolog zu der Sequenz

TTTA$^C_T$ ATTTTC oder

TATGATAATGAG oder

TGG(N)$_{6-7}$GCCAA

ist und ein ineffizientes Selektionssystem enthält.

Bevorzugte Bindeproteine sind der Nuclearfaktor III (NF III, Nature 332 [1986] 656), Nuclearfaktor I (NF I, EMBO J. 6 [1987] 161-168) und das zelluläre muARS-Bindeprotein, ein Säugerprotein, welches aus der Nicht-Histonproteinfraktion von Säuger-Zellkernen isoliert werden kann und an die Sequenz

TTTA$_\mathrm{T}^\mathrm{C}$ ATTTTC

bindet.

NF I bindet beispielsweise an die Sequenz

TGG(N)$_{6-7}$ GCCAA.

NF III bindet beispielsweise an die Sequenz

TATGATAATGAG.

Wenn die erste Consensussequenz und die zweite Consensussequenz nur je einmal im Vektor enthalten sind, so beträgt ihr Abstand vorteilhaft 20 - 150 Basenpaare (bp) mit beliebiger Basenfolge. Es hat sich jedoch als vorteilhaft erwiesen, eine Basenfolge zu wählen, die in den Säugergenomen nicht natürlicherweise vorkommt. Die Polaritätszuordnung der Consensussequenzen kann willkürlich sein.

Vorteilhaft liegen die erste und/oder die zweite Consensussequenz als Multimere vor. Besonders vorteilhaft hat sich die Verwendung eines 2- bis 6meren erwiesen. Dabei können die wiederholten Sequenzen direkt aneinander gekoppelt sein oder einen Abstand von wenigen bp (z. B. 2 - 5) besitzen. Der Abstand der Multimeren untereinander beträgt vorteilhaft 20 - 150 bp mit beliebiger Basenfolge. Auch hier kann die Polaritätszuordnung beliebig sein.

Zusätzlich enthält der Vektor ein ineffizientes Selektionssystem. Unter einem ineffizienten Selektionssystem versteht man die Kombination aus einem Promotor und einem selektiven Gen, die so ausgewählt sind, daß nach Transfektion des Vektors in die Rezipientenzelle diese den Selektionsdruck durch das entsprechende Selektionsmittel nicht überleben kann, wenn der Vektor nicht repliziert wird. Im nicht amplifizierten Zustand muß also das Produkt des selektiven Gens (Genprodukt) in einer Konzentration entstehen, die kleiner als die Schwellenkonzentration ist. Unter Schwellenkonzentration versteht man die Konzentration, die von diesem Genprodukt gebraucht wird, damit die Zelle überlebt.

Geeignete selektive Gene sind beispielsweise tk (Nature 303 (1983) 442 - 446), neo (J. Mol. Appl. Genet. 1 (1982) 327 - 341), dhfr (P.N.A.S. USA 77 (1980) 4216 - 4220, J. Mol. Biol. 15 (1982) 601 - 621), hgprt (P.N.A.S. USA 78 (1981) 2072 - 2076), aprt oder metallothionein.

Die geeigneten Wirtszellen müssen eine entsprechende Defizienz aufweisen. Dazu sind beispielsweise bei Verwendung von tk als selektivem Gen Maus-LMTK-Zellen (ATCC CCL 1,3), oder tk-defiziente Mastocytomzellen (Maus, Somatic Cell and Molecular Genetics 11 (1985) 467 - 475) geeignet.

Die entsprechenden Selektionsmittel sind dem Fachmann geläufig und sind beispielsweise für tk HAT-Medium, für hgprt 8-Azaguanin oder 6-Thioguanin, für aprt Azaserin und Adenin und für dhfr Aminopterin und Methotrexat.

Als Promotor kann ein schwacher Promotor oder ein nachträglich ineffizient gemachter Promotor verwendet werden. Die Promotorstärke kann beispielsweise durch Einführen von Punktmutationen (Cell 45 [1986] 743-751) oder Deletionsmutagenese (Cell 37 [1984] 253-262) verringert werden. Beispielsweise kann für das tk-Gen eine Deletion der distalen SP-I-Bindungsstelle durch Behandeln mit EcoRI durchgeführt werden (Nucl. Acids Res. 8 [1980] 5949-5964). Außerdem kann die Promotorstärke durch Zugabe von Repressoren vermindert werden (Cell 49, (1987) 603 - 612, EMBO J. 2 (1983) 2229 - 2303, Cell 48 (1987) 555 - 566).

Anstelle eines kompletten bzw. mutierten Promotors kann ebenso ein analog wirkendes System, welches aus einer Polymerase-Bindungsstelle, die eine TATA-Box enthält, verwendet werden. Auch hier muß die Expression des selektiven Gens so geregelt sein, daß das Genprodukt unter Normalbedingungen nur in einer Konzentration entsteht, die unter einer Schwellenkonzentration liegt.

Das zu exprimierende Gen muß im Vektor so eingebaut sein, daß es unter Kontrolle des hierfür vorgesehenen Promotors steht. Außerdem sollte es nicht zwischen die erste und zweite Consensussequenz insertiert sein.

Ein weiterer Gegenstand der Erfindung ist ein Verfahren zur Expression von Proteinen in Säugerzellen dadurch gekennzeichnet, daß ein Vektor, der mindestens eine erste Consensussequenz, welche 10 bis 12 Nukleotide und folgende Sequenz aufweist:

CTCTGAGATC,

CTCTAAGAGGAA oder

CTA$_\mathrm{T}^\mathrm{A}$ GAGA$_\mathrm{CC}^\mathrm{GG}$ AA,

eine zweite Consensussequenz, welche 9 bis 12 Nukleotide aufweist, wovon mindestens 9 Adenin und/oder Thymidin sind, und ein Bindeprotein binden kann, sowie die folgende Nukleotidfolge aufweist:

TGG(N)$_{6-7}$ GCCAA,

wobei der Abstand zwischen erster und zweiter Consensussequenz 20 bis 150 Basenpaare beträgt und ein ineffizientes Selektionssystem enthält, nach bekanntem Verfahren in Säugerzellen eingeführt wird, die Zellen vermehrt werden und das entstandene Protein isoliert wird.

Vorzugsweise werden die weiter oben beschriebenen Vektoren verwendet.

Bei dem erfindungsgemäßen Verfahren ist es möglich, durch Variation der ersten und/oder zweiten Consensussequenz im erfindungsgemäß beschriebenen Umfang mit geringer oder hoher Kopienzahl des Vektors zu arbeiten.

So ist es beispielsweise vorteilhaft, wenn ein toxisches Protein exprimiert werden soll, mit einer geringen Kopienzahl zu arbeiten.

Soll dagegen im batch-Verfahren gearbeitet werden, so wird ein Vektor ausgewählt, der sich in hoher Kopienzahl amplifiziert.

Die gentechnologischen Standardprozeduren, wie Transformation, Klonierung und Restriktion, wurden, wenn nichts anderes erwähnt, analog Maniatis, T., Fritsch, E.F., and Sambrook, J. (1982) "Molecular Cloning", Cold Spring Harbor Laboratory, Cold Spring Harbor, NY 11724, durchgeführt. Molekularbiologische Reagenzien wurden nach Vorschrift der Hersteller eingesetzt.

Die Erfindung wird durch die folgenden Beispiele und Abbildungen weiter erläutert.

Es zeigen:

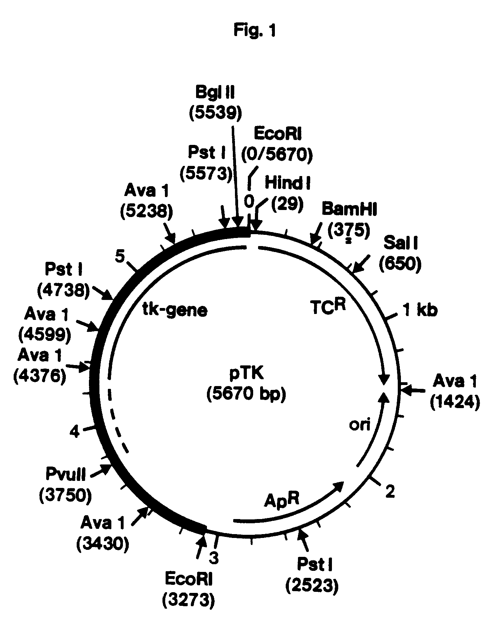

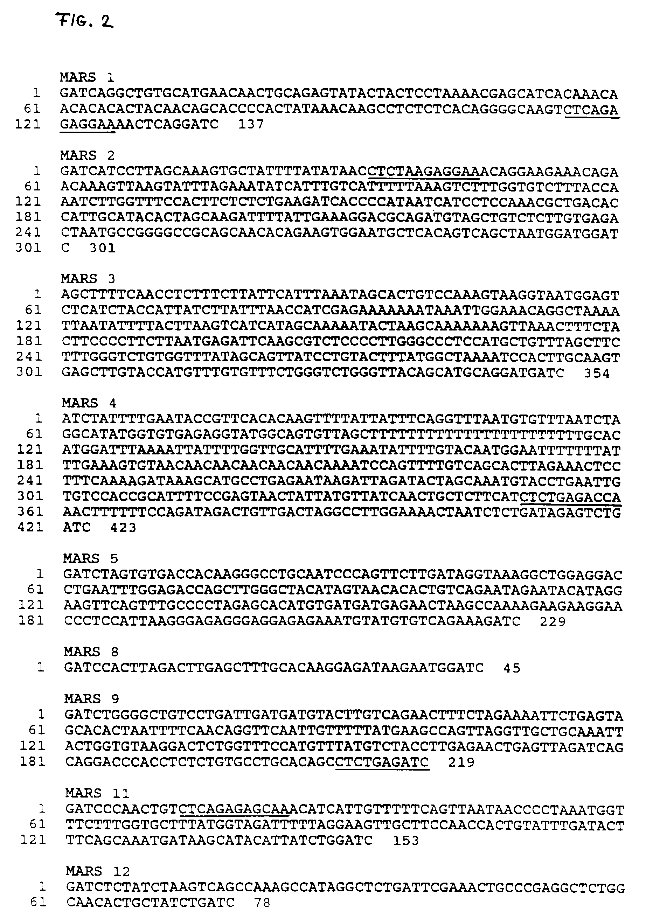

Fig. 1 Vektor ptk

**Beispiel 1**

**Konstruktion eines Vektors mit einem ineffizienten Selektionssystem**

Ein 2.487 bp langes EcoRI-Fragment aus HSV1-DNA (Sequenz Nucl. Acids Res. 8 [1980] 5949-5964), welches das komplette Herpes-Simplex-Virus Thymidinkinasegen (HSV1-TK-Gen) und 80 bp der tk-Promotorregion enthält, wird in der zweiten distalen Regulationssequenz trunkiert (Cell 37 [1984] 253-262).

Dieses Fragment wird in den Vektor pBR327 (Gene 9 [1980] 287-305), der ebenfalls mit EcoRI behandelt wurde, einligiert. Dabei entsteht das Plasmid ptk (DSM 4203P) (Fig. 1). Dieses wird in E. coli HB 101 (DSM 1607) transformiert. Die ampicillin- und tetracyclinresistenten Kolonien werden isoliert und hieraus Plasmid-DNA präpariert. Das Plasmid ist dadurch charakterisiert, daß bei seiner Spaltung mit PstI Fragmente der Größe 2,7 kb, 2,2 kb und 0,75 kb entstehen.

Nach Transfektion von LMTK$^-$-Zellen (ATCC CCL 1.3) mit diesem Plasmid analog Beispiel 3 sterben die Rezipientenzellen unter HAT-Selektionsbedingungen im Verlauf von etwa zwei Wochen ab.

**Beispiel 2**

**Konstruktion von Vektoren, die eine erste und eine zweite Consensussequenz besitzen**

Das durch Spaltung mit BamHI linearisierte Plasmid ptk wird durch Zugabe von Nuclease S1 an beiden Enden glatt gemacht. Dieses Fragment wird mit einem Oligonucleotid aus Tabelle 2 gemischt und durch Zugabe von Ligase ligiert. Nach Klonierung in E. coli HB101 wird die Plasmid-DNA isoliert und über Vergleich von Restriktionsfragmenten mit Längenstandards in der Gelelektrophorese identifiziert.

Das 219 bp lange muARS 9-Fragment wird mit Sau 3A in zwei Subfragmente geschnitten. Das 45 bp-Subfragment wird isoliert und in die BamHI-site von ptk eingesetzt.

Das rekombinante Plasmid (charakterisiert durch Fragmente der Längen 2397, 2623 und 695 bp nach Spaltung mit EcoRI und SalI) ist ebenso aktiv wie das Plasmid, welches das komplette muARS 9-Fragment enthält. Das 45 bp-Subfragment wird tetramerisiert und als Tandem in den glatt gemachten und mit BamHI linearisierten Vektor ptk einligiert. Der Vektor ist dadurch charakterisiert, daß bei seiner Spaltung mit EcoRI und SalI Fragmente der Größe 2397, 2623 und 830 bp entstehen.

**Beispiel 3**

**Transfektion von Maus-LMTK$^-$-Zellen**

Die Transfektion wird, wie in Graham, Virology 52 (1973) 456-467 und Wigler, Proc. Natl. Acad. Sci. USA 76 (1979) 1373-1376 beschrieben, durchgeführt. Die Zellen werden 1 Tag vor der Transfektion in einer Dichte von $3 \times 10^4$ Zellen pro 6 cm Petrischale ausgesät.

In einer Petrischale (6 cm Durchmesser) werden 1 $\mu$g steriler supercoil-Plasmid-DNA auf 225 $\mu$l durch Zugabe von 1 mmol/l Tris-HCl, pH 8,1, 0,1 mmol/l EDTA verdünnt. Anschließend werden 50 $\mu$l 2,5 mol/l Calciumchlorid zugegeben. Anschließend werden 250 $\mu$l HEPES-buffered saline (28 mmol/l NaCl, 50 mmol/l HEPES pH 7,1, 1,5 mmol/l $Na_2HPO_4$) unter Rühren tropfenweise zugegeben und bei Raumtemperatur für 30 min inkubiert.

Die Probe wird zu 5 ml der Zellkultur in einer Petrischale zugegeben und 8 Stunden bei 37 °C inkubiert. Anschließend wird das Medium erneuert und nochmals für 24 Stunden inkubiert. Dieses Medium enthält zusätzlich 15 $\mu$g/ml Hypoxanthin, 0,2 $\mu$g/ml Aminopterin und 5 $\mu$g/ml Thymidin (HAT). In Abständen von 3 Tagen werden die Zellen mit frischem HAT-Medium versorgt. Nach 14 und 21 Tagen sind HAT-resistente Kolonien sichtbar. Die HAT-resistenten Kolonien werden isoliert und angezüchtet.

Die Zellzüchtung erfolgt in Dulbecco's modified Eagle's Minimal medium (Gibco), welches zusätzlich 10 % FKS, 100 $\mu$g/ml Penicillin und 100 $\mu$g/ml Streptomycin enthält.

## Beispiel 4

### Vergleich der Plasmidamplifikation in LMTK⁻-Zellen

Bei der Transfektion von Maus-LMTK⁻-Zellen mit Vektoren nach Beispiel 1 und 2, welche verschiedene muARS enthalten, und anschließender Kultur der Zellen über 60 Tage werden die Replikationsraten (Kopienzahl pro Zelle) von Tabelle I erhalten.

Die Bestimmung der Replikation erfolgt über die Bestimmung der Anzahl der replizierten Plasmide pro Zelle. Hierzu wird nach Zellzüchtung (Beispiel 3) die niedermolekulare DNA nach Hirt (J. Mol. Biol. [1967] 26, 365-369) extrahiert und die Mengen nach Gelelektrophorese visuell bestimmt.

Tabelle I

| muARS-DNA | Länge des DNA-Fragments (bp) | Kopienzahl pro Zelle |
|---|---|---|
| keine muARS-DNA | - | 0 |
| muARS-1 | 137 | 530 ± 270 |
| muARS-2 | 301 | 70 ± 30 |
| muARS-3 | 354 | 3000 ± 2700 |
| muARS-4 | 423 | 1500 ± 2500 |
| muARS-5 | 229 | 170 ± 70 |
| muARS-8 | 45 | 110 ± 20 |
| muARS-9 | 219 | 400 ± 360 |
| muARS-10 | 2500 | 75 ± 45 |
| muARS-11 | 153 | 205 ± 85 |
| muARS-12 | 78 | 555 ± 475 |

## Beispiel 5

### Expression von tPA in Maus-Fibroblasten (LMTK⁻)

Wie in Beispiel 2 beschrieben, wird in dem mit BamHI linearisierten Vektor ptk das Oligonukleotid muARS-4 ligiert und in E. coli kloniert.

Anschließend wird mit SalI geschnitten und die aus dem Vektor pKCR-tPA$_1$ mit AatII und SalI (partiell) herausgeschnittene tPA-Expressionskassette einligiert. Nach Klonierung in E. coli wird das Plasmid ptktPA erhalten, welches dadurch charakterisiert ist, daß durch Spaltung mit der Restriktions-Endonuklease EcoRI 6 Fragmente entstehen [0,4 kb, 1.1 kb, 1,5 kb (Doppelbande), 2,5 kb und 3,6 kb].

Mit diesen Plasmiden werden, wie in Beispiel 3 beschrieben, Maus-LMTK⁻-Zellen transfektiert. Nach 14 Tagen werden 30 Kolonien (erkennbar als Zellhaufen), isoliert und 3 Wochen weitergezüchtet. Von diesen 30 Kolonien ergaben 10 Klone eine Ausbeute von 6 - 8 $\mu$g tPA/10$^6$ Zellen/24 h/ml, 10 Klone eine Menge von 3 - 4 $\mu$g tPA und 10 Klone eine Menge von 1 - 2 $\mu$g tPA.

## Patentansprüche

1. Vektor zur Expression von heterologen Proteinen in Säugerzellen, dadurch gekennzeichnet, daß er mindestens (a) aus einer ersten Consensussequenz, welche 10 bis 12 Nukleotide und folgende Sequenz aufweist:
CTCTGAGATC,

CTCTAAGAGGAA oder

CTC$^A_T$ GAGA$^{GG}_{CC}$ AA

und (b) einer zweiten Consensussequenz, welche 9 bis 12 Nukleotide aufweist, wovon mindestens 9 Adenin und/oder Thymidin sind, und ein Bindeprotein binden kann, sowie die folgende Nukleotidfolge aufweist:

TGG(N)$_{6-7}$GCCAA,

wobei der Abstand zwischen erster und zweiter Consensussequenz 20 bis 150 Basenpaare betragt, vor der ersten oder hinter der zweiten Consensussequenz eine DNA-Sequenz, welche für ein heterologes Protein kodiert, aufweist

und (c) aus einem ineffizienten Selektionssystem besteht.

2. Vektor nach Anspruch 1, dadurch gekennzeichnet, daß das Selektionssystem aus einem trunkierten tk-Promotor und dem tk-Gen besteht.

3. Vektor nach Ansprüche 1 oder 2, dadurch gekennzeichnet, daß er für das zu exprimierende Gen geeignete Promotor- und und Terminatorsequenzen enthalt.

4. Vektor nach Anspruch 1, dadurch gekennzeichnet, daß die erste Consensussequenz folgende Nukleoidsequenz aufweist:

CTC$^A_T$ GAGA$^{GG}_{CC}$ AA

5. Verfahren zur Herstellung eines Vektors zur Expression von heterologen Proteinen in Säugerzellen, dadurch gekennzeichnet, daß nach bekannten Methoden oberhalb (5') oder unterhalb (3') von dem zu exprimierenden Gen (a) eine erste Consensussequenz, welche 10 bis 12 Nukleotide und eine der folgenden Sequenzen:

CTCTGAGATC,

CTCTAAGAGGAA oder

CTC$^A_T$ GAGA$^{GG}_{CC}$ AA

aufweist, und (b) eine zweite Consensussequenz, welche 9 bis 12 Nukloeotide aufweist, wovon mindestens 9 Adenin und/oder Thymidin sind, und ein Bindeprotein binden kann, sowie die folgende Nukleotidfolge aufweist:

TGG(N)$_{6-7}$GCCAA,

ein ineffizientes Selektionssystem und vor der ersten und hinter der zweiten Consensussequenz eine DNA-Sequenz enthält, welche für ein heterologes Protein codiert, eingefügt werden.

6. Verfahren nach Anspruch 5, dadurch gekennzeichnet, daß der Abstand zwischen erster und zweiter Consensussequenz 20 bis 150 Basenpaare beträgt.

7. Verfahren nach Anspruch 5 oder 6, dadurch gekennzeichnet, daß der Vektor als ineffizientes Selektionssystem einen trunkierten tk-Promotor und das tk-Gen enthält.

8. Verfahren nach den Ansprüchen 5 bis 7, dadurch gekennzeichnet, daß als erste Consensussequenz CTC$^A_T$ GAGA$^{GG}_{CC}$ AA verwendet wird.

9. Verfahren zur Herstellung von Proteinen, dadurch gekennzeichnet, daß ein Vektor verwendet wird, der mindestens (a) eine erste Consensussequenz, welche 10 bis 12 Nukleotide und folgende Sequenz aufweist:

CTCTGAGATC,

CTCTAAGAGGAA oder

CTC$^A_T$ GAGA$^{GG}_{CC}$ AA,

(b) eine zweite Consensussequenz, welche 9 bis 12 Nukleotide aufweist, wovon mindestens 9 Adenin und/oder Thymidin sind, und ein Bindeprotein binden kann, sowie die folgende Nukleotidfolge aufweist:

TGG(N)$_{6-7}$GCCAA,

(c) ein ineffizientes Selektionssystem und (d) vor der ersten oder hinter der zweiten Consensussequenz die zu exprimierende DNA-Sequenz aufweist.

**10.** Verfahren nach Anspruch 9, dadurch gekennzeichnet, daß der Abstand zwischen erster und zweiter Consensussequenz 20 bis 150 Basenpaare beträgt.

**11.** Verfahren nach Anspruch 9 oder 10, dadurch gekennzeichnet, daß als erste Consensussequenz die Sequenz
$CTC^A_T$ $GAGA^{GG}_{CC}$ $AA$
verwendet wird.

**12.** Verfahren nach den Ansprüchen 9 bis 11, dadurch gekennzeichnet, daß der Vektor als ineffizientes Selektionssystem einen trunkierten tk-Promotor und das tk-Gen enthält.

**13.** Verfahren nach den Ansprüchen 9 bis 12, dadurch gekennzeichnet, daß während der Fermentation ein Selektionsmittel zugegeben wird.

**Claims**

**1.** Vector for the expression of heterologous proteins in mammalian cells, characterised in that it consists at least (a) of a first consensus sequence which has 10 to 12 nucleotides and the following sequence:
CTCTGAGATC,
CTCTAAGAGGAA or
$CTC^A_T$ $GAGA^{GG}_{CC}$ $AA$
and (b) of a second consensus sequence which has 9 to 12 nucleotides, of which at least 9 are adenine and/or thymidine, and can bind a binding protein, as well as has the following nucleotide sequence:
$TGG(N)_{6-7}GCCAA$,
whereby the distance between first and second consensus sequence amounts to 20 to 150 base pairs, before the first or after the second consensus sequence has a DNA sequence which codes for a heterologous protein and (c) of an inefficient selection system.

**2.** Vector according to claim 1, characterised in that the selection system consists of a truncated tk promotor and the tk gene.

**3.** Vector according to claim 1 or 2, characterised in that it contains promotor and terminator sequences suitable for the gene to be expressed.

**4.** Vector according to claim 1, characterised in that the first consensus sequence has the following nucleotide sequence:
$CTC^A_T$ $GAGA^{GG}_{CC}$ $AA$.

**5.** Process for the production of a vector for the expression of heterologous proteins in mammalian cells, characterised in that, according to known methods, there are introduced above (5') and below (3') from the gene to be expressed (a) a first consensus sequence which has 10 to 12 nucleotides and one of the following sequences:
CTCTGAGATC,
CTCTAAGAGGAA or
$CTC^A_T$ $GAGA^{GG}_{CC}$ $AA$
and (b) a second consensus sequence which has 9 to 12 nucleotides, of which at least 9 are adenine and/or thymidine, and can bind a binding protein, as well as has the following nucleotide sequence:
$TGG(N)_{6-7}GCCAA$,
an inefficient selection system and, before the first and after the second consensus sequence, contains a DNA sequence which codes for a heterologous protein.

**6.** Process according to claim 5, characterised in that the distance between first and second consensus sequence amounts to 20 to 150 base pairs.

**7.** Process according to claim 5 or 6, characterised in that, as inefficient selection system, the vector contains a truncated tk promotor and the tk gene.

**8.** Process according to claims 5 to 7, characterised in that $CTC_T^A$ $GAGA_{CC}^{GG}$ AA is used as first consensus sequence.

**9.** Process for the production of proteins, characterised in that a vector is used which contains at least (a) a first consensus sequence which has 10 to 12 nucleotides and the following sequence:
CTCTGAGATC,
CTCTAAGAGGAA or
$CTC_T^A$ $GAGA_{CC}^{GG}$ AA,
(b) a second consensus sequence which has 9 to 12 nucleotides, of which at least 9 are adenine and/or thymidine, and can bind a binding protein, as well as has the following nucleotide sequence:
$TGG(N)_{6-7}GCCAA$,
(c) an inefficient selection system and (d) has the DNA sequence to be expressed before the first or after the second consensus sequence.

**10.** Process according to claim 9, characterised in that the distance between first and second consensus sequence amounts to 20 to 150 base pairs.

**11.** Process according to claim 9 or 10, characterised in that the sequence $CTC_T^A$ $GAGA_{CC}^{GG}$ AA is used as first consensus sequence.

**12.** Process according to claims 9 to 11, characterised in that the vector contains a truncated tk promotor and the tk gene as inefficient selection system.

**13.** Process according to claims 9 to 12, characterised in that a selection agent is added during the fermentation.

**Revendications**

**1.** Vecteur d'expression de protéines hétérologues dans des cellules de mammifères, caractérisé en ce qu'il présente au moins (a) une première séquence consensus, qui comprend 10 à 12 nucléotides et la séquence suivante :
CTCTGAGATC,
CTCTAAGAGGAA
ou
$CTC_T^A$ $GAGA_{CC}^{GG}$ AA
et (b) une seconde séquence consensus, qui comprend 9 à 12 nucléotides, dont au moins 9 sont l'adénine et/ou la thymidine, et peut fixer une protéine de liaison, et comprend la séquence de nucléotide suivante :
$TGG(N)_{6-7}GCCAA$,
la distance entre la première et la seconde séquence consensus étant de 20 à 150 paires de bases, et qui comprend, avant la première ou après la seconde séquence consensus, une séquence d'ADN codant pour une protéine hétérologue, et (c) un système de sélection inefficace.

**2.** Vecteur selon la revendication 1, caractérisé en ce que le système de sélection est constitué d'un promoteur tk tronqué, et du gène tk.

**3.** Vecteur selon la revendication 1 ou 2, caractérisé en ce que le gène à exprimer contient des séquences de promoteur et de terminateur appropriées.

**4.** Vecteur selon la revendication I, caractérisé en ce que la première séquence consensus présente la séquence de nucléotide suivante :
$CTC_T^A$ $GAGA_{CC}^{GG}$ AA

**5.** Procédé permettant la construction d'un vecteur d'expression de protéines hétérologues dans des cellules de mammifères, caractérisé en ce que selon des méthodes connues, on insère, en amont (en 5') ou en aval (en 3') du gène à exprimer, (a) une première séquence consensus, qui comprend 10 à 12 nucléotides et la séquence suivante :
CTCTGAGATC,

CTCTAAGAGGAA

ou

$\mathrm{CTC^{A}_{T}\,GAGA^{GG}_{CC}\,AA}$

et (b) une seconde séquence consensus, qui comprend 9 à 12 nucléotides, dont au moins 9 sont l'adénine et/ou la thymidine, et peut fixer une protéine de liaison, et comprend la séquence de nucléotide suivante :

$TGG(N)_{6-7}GCCAA$,

et en ce que le vecteur contient un système de sélection inefficace et avant la première ou après la seconde séquence consensus, une séquence d'ADN codant pour une protéine hétérologue.

6. Procédé selon la revendication 5, caractérisé en ce que la distance entre la première et la seconde séquence consensus est de 20 à 150 paires de bases.

7. Procédé selon la revendication 5 ou 6, caractérisé en ce que le vecteur contient, comme système de sélection inefficace, un promoteur tk tronqué et le gène tk.

8. Procédé selon les revendications 5 à 7, caractérisé en ce que comme première séquence consensus, on utilise

$\mathrm{CTC^{A}_{T}\,GAGA^{GG}_{CC}\,AA}$

9. Procédé de préparation de protéines, caractérisé en ce qu'on utilise un vecteur qui présente au moins (a) une première séquence consensus, qui comprend 10 à 12 nucléotides et la séquence suivante :

CTCTGAGATC,

CTCTAAGAGGAA

ou

$\mathrm{CTC^{A}_{T}\,GAGA^{GG}_{CC}\,AA}$,

(b) une seconde séquence consensus, qui comprend 9 à 12 nucléotides, dont au moins 9 sont l'adénine et/ou la thymidine, et qui peut fixer une protéine de liaison, ainsi que la séquence nucléotide suivante :

$TGG(N)_{6-7}GCCAA$,

(c) un système de sélection inefficace et (d) avant la première ou après la seconde séquence consensus, la séquence d'ADN à exprimer.

10. Procédé selon la revendication 9, caractérisé en ce que la distance entre la première et la seconde séquence consensus est de 20 à 150 paires de bases.

11. Procédé selon la revendication 9 ou 10, caractérisé en ce que comme première séquence consensus, on utilise la séquence

$\mathrm{CTC^{A}_{T}\,GAGA^{GG}_{CC}\,AA}$

12. Procédé selon les revendications 9 à 11, caractérisé en ce que le vecteur contient, comme système de sélection inefficace, un promoteur tk tronqué et le gène tk.

13. Procédé selon les revendications 9 à 12, caractérisé en ce que pendant la fermentation, on ajoute un agent de sélection.

Fig. 1

FIG. 2

MARS 1
GATCAGGCTGTGCATGAACAACTGCAGAGTATACTACTCCTAAAACGAGCATCACAAACA
ACACACACTACAACAGCACCCCACTATAAACAAGCCTCTCTCACAGGGGCAAGTCTCAGA
GAGGAAAACTCAGGATC  137

MARS 2
GATCATCCTTAGCAAAGTGCTATTTTATATAACCTCTAAGAGGAAACAGGAAGAAACAGA
ACAAAGTTAAGTATTTAGAAATATCATTTGTCATTTTTAAAGTCTTTGGTGTCTTTACCA
AATCTTGGTTTCCACTTCTCTCTGAAGATCACCCCATAATCATCCTCCAAACGCTGACAC
CATTGCATACACTAGCAAGATTTTATTGAAAGGACGCAGATGTAGCTGTCTCTTGTGAGA
CTAATGCCGGGGCCGCAGCAACACAGAAGTGGAATGCTCACAGTCAGCTAATGGATGGAT
C  301

MARS 3
AGCTTTTCAACCTCTTTCTTATTCATTTAAATAGCACTGTCCAAAGTAAGGTAATGGAGT
CTCATCTACCATTATCTTATTTAACCATCGAGAAAAAAATAAATTGGAAACAGGCTAAAA
TTAATATTTTACTTAAGTCATCATAGCAAAAATACTAAGCAAAAAAAGTTAAACTTTCTA
CTTCCCCTTCTTAATGAGATTCAAGCGTCTCCCCTTGGGCCCTCCATGCTGTTTAGCTTC
TTTGGGTCTGTGGTTTATAGCAGTTATCCTGTACTTTATGGCTAAAATCCACTTGCAAGT
GAGCTTGTACCATGTTTGTGTTTCTGGGTCTGGGTTACAGCATGCAGGATGATC  354

MARS 4
ATCTATTTTGAATACCGTTCACACAAGTTTTATTATTTCAGGTTTAATGTGTTTAATCTA
GGCATATGGTGTGAGAGGTATGGCAGTGTTAGCTTTTTTTTTTTTTTTTTTTTTTTTGCAC
ATGGATTTAAAATTATTTTGGTTGCATTTTGAAATATTTTGTACAATGGAATTTTTTTAT
TTGAAAGTGTAACAACAACAACAACAACAAAATCCAGTTTTGTCAGCACTTAGAAACTCC
TTTCAAAAGATAAAGCATGCCTGAGAATAAGATTAGATACTAGCAAATGTACCTGAATTG
TGTCCACCGCATTTTCCGAGTAACTATTATGTTATCAACTGCTCTTCATCTCTGAGACCA
AACTTTTTTCCAGATAGACTGTTGACTAGGCCTTGGAAAACTAATCTCTGATAGAGTCTG
ATC  423

MARS 5
GATCTAGTGTGACCACAAGGGCCTGCAATCCCAGTTCTTGATAGGTAAAGGCTGGAGGAC
CTGAATTTGGAGACCAGCTTGGGCTACATAGTAACACACTGTCAGAATAGAATACATAGG
AAGTTCAGTTTGCCCCTAGAGCACATGTGATGATGAGAACTAAGCCAAAAGAAGAAGGAA
CCCTCCATTAAGGGAGAGGGAGGAGAGAAATGTATGTGTCAGAAAGATC  229

MARS 8
GATCCACTTAGACTTGAGCTTTGCACAAGGAGATAAGAATGGATC  45

MARS 9
GATCTGGGGCTGTCCTGATTGATGATGTACTTGTCAGAACTTTCTAGAAAATTCTGAGTA
GCACACTAATTTTCAACAGGTTCAATTGTTTTTATGAAGCCAGTTAGGTTGCTGCAAATT
ACTGGTGTAAGGACTCTGGTTTCCATGTTTATGTCTACCTTGAGAACTGAGTTAGATCAG
CAGGACCCACCTCTCTGTGCCTGCACAGCCTCTGAGATC  219

MARS 11
GATCCCAACTGTCTCAGAGAGCAAACATCATTGTTTTTCAGTTAATAACCCCTAAATGGT
TTCTTTGGTGCTTTATGGTAGATTTTTAGGAAGTTGCTTCCAACCACTGTATTTGATACT
TTCAGCAAATGATAAGCATACATTATCTGGATC  153

MARS 12
GATCTCTATCTAAGTCAGCCAAAGCCATAGGCTCTGATTCGAAACTGCCCGAGGCTCTGG
CAACACTGCTATCTGATC  78